# EUROPÄISCHE PATENTSCHRIFT

(11) **EP 2 809 163 B1**
(45) Veröffentlichungstag und Bekanntmachung des Hinweises auf die Patenterteilung: **11.03.2020**
(21) Anmeldenummer: 13705404.5
(22) Anmeldetag: 24.01.2013
(51) Int. Cl.: C12P 19/04, A21D 8/04, C12N 1/20

(54) **EINSATZ VON ESSIGSÄUREBAKTERIEN ZUR HERSTELLUNG VON BACKWAREN**
USE OF ACETIC ACID BACTERIA FOR THE PREPARATION OF BAKERY PRODUCTS
UTILISATION DE BACTÉRIES ACIDO-ACÉTIQUES POUR LA PRÉPARATION DE PRODUITS DE BOULANGERIE

(30) Priorität: 01.02.2012 DE 102012100834
(43) Veröffentlichungstag der Anmeldung: 10.12.2014
(73) Patentinhaber: Ernst Böcker GmbH & Co. KG, 32427 Minden (DE)
(72) Erfinder: BRANDT, Markus, 32423 Minden (DE)
(74) Vertreter: Fleuchaus, Andrea
(86) Internationale Anmeldenummer: PCT/EP2013/051284
(87) Internationale Veröffentlichungsnummer: WO 2013/113602

(56) Entgegenhaltungen:
- EP-A1- 0 903 082
- DE-A1- 3 541 292
- DE-T2- 3 850 477
- GB-A- 1 570 487
- DATABASE WPI Week 2001 Thomson Scientific, London, GB; AN 2001-545016 XP002716087, & JP 2001 204376 A 31. Juli 2001 (2001-07-31)

## Beschreibung

Die vorliegende Erfindung betrifft eine nicht-entkeimte Getreide- und/oder Pseudogetreidemaische unter Einsatz von Essigsäurebakterien bei der Herstellung von Backwaren. Essigsäurebakterien bilden mehrere Stoffwechselprodukte von backtechnologischer Bedeutung, wie z.B. Essigsäure, Gluconsäure und Exopolysaccharide. Allerdings war der Einsatz von Essigsäurebakterien in der Anwendung einer Teigfermentationen bisher nicht möglich, da die Essigsäurebakterien zum Wachstum Sauerstoff brauchen und das Einbringen von Sauerstoff, sofern Teige mit Sauerstoff oder Luft versetzt werden oder gerührt werden, das Wachstum von zahlreichen Kontaminanten aus dem Mehl erheblich fördert. Solche Kontaminanten zeigen deutlich höhere Wachstumsraten als die langsam wachsenden Essigsäurebakterien, wodurch die Essigsäurebakterien faktisch überwachsen werden. Der Einsatz von Essigsäurebakterien bei der Herstellung von Backwaren ist daher derzeit nicht möglich.

Die vorliegende Erfindung macht es sich zur Aufgabe diesen Missstand zu beheben und den Einsatz von Essigsäurebakterien in nicht-entkeimter Getreide- und/oder Pseudogetreidemaische zur Herstellung von Backwaren zu ermöglichen.

Essigsäurebakterien sind in der Natur weit verbreitet und sind überall dort zu finden, wo aus Pflanzen zuckerreiche Säfte auftreten und gleichzeitig Sauerstoff verfügbar ist. Sie sind säuretolerant, wachsen noch unterhalb von pH 3,7 und sind obligat aerob. Industrielle Bedeutung besitzen Sie für die Herstellung Essig aus Wein Branntwein und anderen alkoholischen Getränken. Aufgrund ihrer hohen Alkoholtoleranz haben sie in diesem Ökosystem kaum Konkurrenten. Die Essigsäurebakterien wurden ursprünglich von Bejerinck als *Acetobacter aceti* mit nur einer Spezies beschrieben. Allen Essigsäurebakterien ist es gemeinsam aus Zuckern oder Alkohol durch unvollständige Oxidation Säuren zu bilden. Die Art des Stoffwechselproduktes hängt entscheidend von der Spezies bzw. dem angebotenen Substrat ab.

Die bekannten Gattungen der Essigsäurebakterien sind: *Acetobacter, Gluconobacter, Acidomonas, Kozakia, Neoasaia, Asaia, Swaminathania, Saccharibacter,* sowie die Gattung *Frateuria,* die zu den "Pseudo-Essigsäurebakterien" gezählt wird. *Gluconobacter* ist im Gegensatz zu *Acetobacter* nicht in der Lage Essigsäure zu Kohlendioxid und Wasser zu oxidieren. Ferner ist *Gluconobacter* in der Lage, eine Vielzahl organischer Substanzen, wie Zucker, Alkohole und Polyole schnell und unvollständig mittels membranständiger Dehydrogenasen zu oxidieren. Zahlreiche *Gluconobacter-Stämme* bevorzugen D-Mannit als Kohlenstoff-Quelle, sie wachsen aber auch gut mit D-Sorbit, Glycerin, D-Fructose und D-Glucose und zahlreichen anderen Zuckern oder Alkoholen.
*Gluconobacter oxydans* z.B. besitzt für die Oxidation von organischen Substanzen zwei alternative Enzymsysteme: zum einen die membrangebundenen Dehydrogenasen, die entweder Pyrroloquinolin Chinon (PQQ) oder ein kovalent gebundenes Flavinadenindinukleotid (FAD) als Kofaktor besitzen und zum anderen die cytoplasmatischen NAD⁺(P⁺)-abhängigen Dehydrogenasen. Die membrangebundenen Enzyme dienen der direkten Substratoxidation, wobei die Elektronen zur Energiegewinnung durch Übertragung auf Ubichinon in die Atmungskette eingeschleust werden. Die aktiven Zentren der Dehydrogenasen liegen im periplasmatischen Raum, die Produkte werden über Poren der äußeren Membran der Zellwand ausgeschleust und akkumulieren im Medium. Dieses Enzymsystem ist für die schnelle Produktion biotechnologisch wichtiger Substanzen verantwortlich. Für die cytosolischen Enzyme, die an den Biomasseerträgen der Zelle beteiligt sind, ist hingegen die Aufnahme der Substrate in die Zelle notwendig. Im Gegensatz zu den membrangebundenen Dehydrogenasen katalysieren die im Cytoplasma lokalisierten Dehydrogenasen auch die reversiblen Reaktionen. Die entstehenden Intermediate werden mittels substratspezifischer Kinasen phosphoryliert und über den Pentosephosphat-Weg oder den Entner-Doudoroff-Weg metabolisiert. So setzt G. *oxydans* z.B. das Substrat Glucose bevorzugt zu Gluconsäure um und bildet aus Mannitol Fructose und Exopolysacchariden (EPS) (De Muynck, C., et al., (2007). Crit Rev Biotechnol 27: 147-71).

Diese und andere Stoffwechselprodukte der Essigsäurebakterien bzw. der *Gluconobacter* hätten backtechnologische Bedeutung, denn durch EPS kann eine Verbesserung der Teig- und somit der Verarbeitungseigenschaften sowie der Brotqualität in Bezug auf Textur, Volumen und auch Haltbarkeit bewirkt werden.

Solche von Essigsäurebakterien gebildeten und für die Backtechnologie bedeutsamen Stoffwechelprodukte, wie z.B. Gluconsäure, Essigsäure und Exopolysaccharide etc., können zwar als zu isolierende Einzelsubstanz biotechnologisch gewonnen und zugesetzt werden, sind dann jedoch ein zulassungsbedürftiger Zusatzstoff (z.B. E574 Gluconsäure, E575 Glucono-delta-lacton, E260 Essigsäure). In der heutigen Zeit möchte der Verbraucher aber auf künstliche Zusatzstoffe verzichten und so sind natürlich fermentierte Produkte hier eine Möglichkeit diesem Bedarf gerecht zu werden.

Derzeit sind z.B. aus EP 0790003B1 oder EP 1283011B1 Verfahren bekannt, die positiven Eigenschaften von Exopolysacchariden (EPS), die von Milchsäurebakterien gebildet wurden, in der Backwarenherstellung einsetzen. Die Bildung von EPS wurde darüber hinaus für eine Vielzahl von Milchsäurebakterien beschrieben (De Vuyst L, Degeest B (1999) Heteropolysaccharides from Lactic Acid Bacteria. FEMS Microbiol Rev 23:153-177), die vielleicht in der Backwarenherstellung einsetzbar sein könnten. Milchsäurebakterien haben hierbei immer den Vorteil, dass sie bei den im Teig üblichen Kulturbedingungen (anaerob, pH kleiner 4) gut wachsen.

Bekannt ist ferner auch, dass z. B. *in situ* gebildetes Dextran von *Leuconostoc mesenteroides* bei der Herstellung von Panettone verwendet wird, was u. a. die Krumenstruktur und die lange Frische des Gebäcks bewirkt. Auch bei der Toast- und Roggenbrotherstellung verbessert Dextran, welches zugesetzt wird, nachweislich die Qualität.

Alternativ lässt sich eine Verbesserung der Teig- und der Verarbeitungseigenschaften sowie der Brotqualität in Bezug auf Textur, Volumen und Haltbarkeit auch durch den Zusatz von Hydrokolloiden bewirken. Diese sind überwiegend pflanzlicher Herkunft, wie Guarkernmehl, Carragenane, Alginate, modifizierte Stärken, Zellulosen und Inulin. Ein Submers-Verfahren zur Herstellung von Bakterienzellulose aus organischen Materialien, bei dem in der Kultur die Belüftung gering gehalten wird, beschreibt bspw. DE 38 50 477 T2. Unter den mikrobiologisch fermentativ erzeugten Hydrokolloiden findet Xanthan aus *Xanthomonas campestris* eine besonders breite Anwendung als Zusatzstoff in unterschiedlichsten Lebensmitteln.

DE 35 41 292 A1 beschreibt ein Verfahren zur Herstellung von Roggenstärke aus einer Wasser/Roggenmehl-Mischung mit säurebildenden Bakterienkulturen, vorzugsweise auf der Basis von *Lactobacillus SPP, Streptokoccus, Acetobacter* oder Sauerteigkulturen. EP 0 903 082 A1 beschreibt ein Verfahren unter Einsatz einer Maische zur Herstellung von geschmackvollen Backwaren. Die hierbei zum Einsatz kommende Maische enthält Mehl und Wasser, sowie zumindest eine säurebildenden Mikroorganismus, u.a. auch eine Mischung von *Lactobacillus* und *Gluconobacter* Spezies ohne Zugabe von Saccharose. AN 2001-545016 & JP 2001 204376 A offenbaren ein Verfahren zur Teigherstellung für Backwaren unter Verwendung von Essigsäurebakterien ohne Zugabe von Saccharose.

Backtechnologisch interessante Stoffwechselprodukte der Essigsäurebakterien stehen allerdings nicht für *in situ* Verfahren zur Verfügung, da Essigsäurebakterien nicht in Getreideteigen wachsen.

Getreidemehle haben üblicherweise eine hohe mikrobielle Beladung im Bereich von 10⁶ KbE/g. Sobald Mehl mit Wasser gemischt wird, steigt der aw-Wert auf fast 1 und mikrobielles Wachstum kann beginnen. Der aw-Wert, der abgeleitet ist von *engl.* activity of water, ist hierbei ein Maß für frei verfügbares Wasser in einem Material. Er ist ein wichtiges und anerkanntes Maß bezüglich der Haltbarkeit von Lebensmitteln, der das Wachstum der Mikroorganismen (Kontaminanten) in dem Lebensmittel beeinflusst, da die meisten Mikroorganismen unterschiedliche Ansprüche an frei verfügbares Wasser haben. Für die meisten Mikroorganismen liegt das Wachstumsoptimum bei einem aw-Wert von 0,98-1.

Bei einem aw-Wert von nahezu 1 wachsen in Getreideteigen zunächst vor allem Enterobakterien, später wird die Fermentation von Milchsäurebakterien dominiert. Die Milchsäurebakterien bilden Milchsäure, was zu einer pH Absenkung führt und zur Folge hat, dass nicht säuretolerante Mikroorganismen (z.B. Enterobakterien oder Bakterien der Gattung *Bacillus)* absterben.

Im weiteren verbrauchen die Milchsäurebakterien den im Teig verbliebenen Restsauerstoff zügig, und schaffen damit ein anaerobes Klima. Spätestens nun sterben auch Essigsäurebakterien ab. Daher wurden Essigsäurebakterien bisher auch nicht in relevanten Keimzahlen aus Sauerteigen isoliert. In der älteren Literatur befindliche Hinweise darauf, sind allein auf die Anwesenheit von Essigsäure zurückgeführt, nicht auf den kulturellen Nachweis von Essigsäurebakterien. Diese Essigsäure wird in Sauerteigen jedoch ausschließlich durch heterofermentative Milchsäurebakterien erzeugt.

Es war die Aufgabe der Erfindung, eine nicht-entkeimte Getreide- und/oder Pseudogetreidemaische zu entwickeln, um Essigsäurebakterien ein Wachstum im Getreideteig zu ermöglichen und so die Stoffwechselprodukte der Essigsäurebakterien in der Backwarentechnologie zur Anwendung zu bringen.

Die vorliegende Erfindung löst die Aufgabe durch eine nicht-entkeimte Getreide- und/oder Pseudogetreidemaische zur Herstellung von Backwaren bei dem Maischen aus pflanzlichen Substraten und Wasser mit Essigsäurebakterien angeimpft wurden, und die Maischen belüftet sind, wobei zum Schutz von Fehlgärungen Saccharose zugesetzt wurde. Durch den Zusatz von Saccharose wird der aw-Wert gesenkt und somit ein Wachstum von Kontaminaten verzögert und sogar ganz verhindert. Durch den Mangel an freiem Wasser, der durch den Zusatz von Saccharose zu dem Substrat gemäß der vorliegenden Erfindung erreicht wird, werden die Wachstumsprozesse von zahlreichen Mikroorganismen - nicht jedoch das Wachstum der zugesetzten Essigsäurebakterien - verlangsamt.
Die vorliegenden Erfindung stellt somit eine nicht-entkeimte Getreide- und/oder Pseudogetreidemaische bereit. Die erfindungsgemäße nicht-entkeimte Getreide- und/oder Pseudogetreidemaische ist dadurch gekennzeichnet, dass die Maische belüftet ist, mit mindestens einer Spezies aus der Gruppe der Essigsäurebakterien bestehend aus Gluconobacter, Gluconacetobacter, Acidomonas, Kozakia, Neoasaia, Asaia, Swaminathania, Saccharibacter, sowie Frateuria angeimpft wurde und die Maische Stoffwechselprodukte, nämlich Essigsäure, Gluconat Gluconsäure und Exopolisaccharide gebildet von mindestens einer Spezies aus der Gruppe der Essigsäurebakterien bestehend aus Gluconobacter, Gluconacetobacter, Acidomonas, Kozakia, Neoasaia, Asaia, Swaminathania, Saccharibacter, sowie Frateuria enthält, wobei diese Stoffwechselprodukte aus einen zugesetzten Anteil von 1,5 - 10 % Saccharose bezogen auf die eingesetzte Mehlmenge in der Maische gebildet wurden und die Maische eine Konzentration der Essigsäurebakterien mit einer Lebendkeimzahl von 10e8 pro ml aufweist.

Die vorliegende Anmeldung stellt auch ein Verfahren zur Anreicherung von Essigsäurebakterien auf nicht-entkeimten Getreide- und/oder Pseudogetreidemaischen bereit, welches die nachfolgend erläuterten Verfahrensschritte umfasst bzw. die aufgelisteten Substrate oder Mirkoorganismen einsetzt.

Zunächst wird gemäß dem anmeldungsgemäßen Verfahren eine Maische aus nicht-entkeimten Getreide oder Pseudogetreide und Wasser zubereitet. Hierzu kommen zu Mehl gemahlene Getreide oder Pseudogetreide ausgewählt aus der Gruppe enthaltend Weizen, Roggen, Gerste, Hafer, Quinoa, Buchweizen, Amaranth, Reis, Mais oder Mischungen aus solchen Getreiden und/oder Pseudogetreiden zum Einsatz.

Die Maische wird anschließend mit mindestens einer Spezies aus der Gruppe der Essigsäurebakterien bestehend aus *Gluconobacter, Acetobacter, Acidomonas,* Gluconacetobacter, *Kozakia, Neoasaia, Asaia, Swaminathania, Saccharibacter,* sowie *Frateuria* oder auch Mischungen derselben angeimpft. Üblicherweise wird hierbei die Maische in dem vorliegenden Verfahren mit ca. 10e2 bis 10e3 Lebendkeimen ausgewählt aus den oben angegebenen Essigsäurebakterien, die in einer Vorkultur - meist übernacht angezogen bzw. kultiviert wurden - angeimpft.

Zusätzlich wird gemäß dem vorliegenden Verfahren die Maische mit Saccharose versetzt. Es werden hierzu zwischen 1,5 - 10 % Saccharose, vorzugsweise 1,5 - 3,%, weiter vorzugsweise 2,0 - 4,5%, weiter vorzugsweise 3,5 - 5,0 %, weiter vorzugsweise 5,0 - 9,5%, weiter vorzugsweise 5 - 7,5%, weiter vorzugsweise 6,0 - 10%, weiter vorzugsweise 2,0 - 8,5% bezogen auf die eingesetzte Mehlmenge zugegeben.

Die vorbereitete, mit Saccharose versetzte und angeimpfte Maische wird dann unter aeroben Kulturbedingungen bei 18-40 °C, vorzugsweise 28-35°C, weiter vorzugsweise 20 - 33°C, weiter vorzugsweise 25 - 38°C, weiter vorzugsweise 22 - 34°C für einen Zeitraum von 10h bis zu 48h, vorzugsweise 10 - 16h, weiter vorzugsweise 12 - 24h, weiter vorzugsweise 12 - 36h, weiter vorzugsweise 24 - 36h, weiter vorzugsweise 20 - 48h, weiter vorzugsweise 18 - 36h inkubiert.

Die Inkubation der Maische ist anmeldungsgemäß abgeschlossen oder abbrechbar sobald wenigstens eine Endkonzentration der Essigsäurebakterien von 10e8 (Lebendkeimzahl) pro ml erreicht wird.

Das anmeldungsgemäße Verfahren stellt somit als Endprodukt eine erfindungsgemäße frische nicht-entkeimte Getreide- und/oder Pseudogetreidemaische bereit, die mindestens eine Spezies aus der Gruppe der Essigsäurebakterien bestehend aus *Gluconobacter, Acetobacter, Acidomonas, Kozakia, Neoasaia, Asaia, Swaminathania, Saccharibacter,* sowie *Frateuria* oder auch Mischungen derselben in einer Konzentration von 10e8 (Lebendkeimzahl) pro ml enthält.

Dieses Endprodukt der nicht-entkeimten Getreide- und/oder Pseudogetreidemaische kann in weiteren Verarbeitungsschritten getrocknet, pasteurisiert oder sterilisiert werden, wobei die Lebendkeimzahl der angereicherten Essigsäurebakterien reduziert wird, aber gleichzeitig die Stoffwechselprodukte der Essigsäurebakterien freigesetzt werden.

Die frische nicht-entkeimten Getreide- und/oder Pseudogetreidemaische kann dann zur *in situ* Produktion von Exopolysaccharide oder anderen Stoffwechselprodukten der Essigsäurebakterien in weiteren Getreidemaischen oder Getreideteigen eingesetzt werden.

Darüber hinaus kann die frische oder weiterbehandelte und z.B. getrocknete nicht-entkeimte Getreide- und/oder Pseudogetreidemaische zur Zugabe bei Produktion von Backwaren und damit zur Verbesserung der zu erzielenden Brotqualität eingesetzt werden.

Es konnte gezeigt werden, dass Brote, die mit der erfindungsgemäß bereitgestellten Getreidemaische, welche eine Lebendkeimzahl von 10e8 Essigsäurenbakterien enthält, und nach dem anmeldungsgemäßen Verfahren hergestellt wurde, verbesserte Volumina und Teigtextur haben. Gleichzeitig ist im Verlauf von mehreren Tagen die Krumenhärte der Brote, die mit Teigen zubereitet nach dem anmeldungsgemäßen Verfahren, welche Essigsäurebakterien enthielten bzw. mit Sauerteig hergestellt wurden, vergleichbar gut. Es zeigte sich auch, dass Brote, die mit Teigen zubereitet nach dem anmeldungsgemäßen Verfahren hergestellt wurden, deutlich länger frisch und weich bleiben, als Brote, die aus üblichen direkt geführten also hefeunterstützten Teigen hergestellt wurden.

### Detaillierte Beschreibung der Figuren

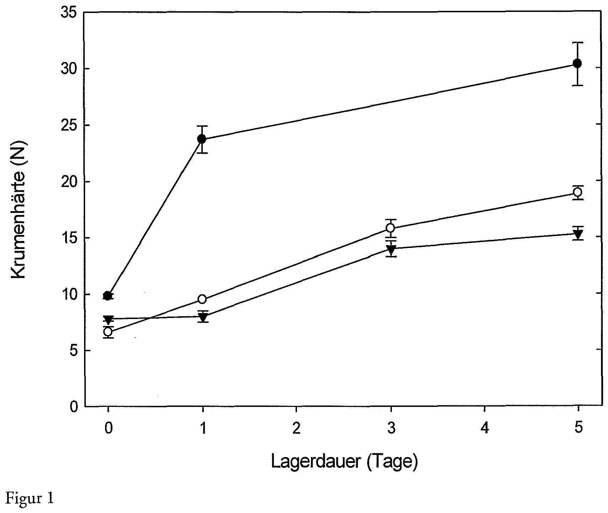

Figur 1 zeigt den zeitlichen Verlauf der Krumenhärte von Weizenbroten, hergestellt in (●) direkter Führung, (○) als Brot mit Sauerteig, (▼) als Brot mit Essigsäurebakterien.

### Beispiel 1 - Vorteig mit Essigsäurebakterien

1 ml gut gewachsene Vorkultur von *Kozakia balinensis* wurde als erster Ansatz im Schikanekolben zu einer Mischung aus 100 g Mehl und 300 g Wasser gegeben und bei 30 °C bei 150 rpm geschüttelt. In einem zweiten Ansatz wurden zusätzlich 40 g Saccharose zu gegeben.
Während in dem Ansatz ohne Saccharose bereits nach kurzer Zeit Milchsäurebakterien auf relevante Keimzahlen angewachsen waren, waren diese bei dem Ansatz mit Saccharosezusatz auch nach 4 Tagen Fermentationszeit kleiner 10⁵ KbE/ml.

Zur Bestimmung langkettiger Fructane aus beiden Ansätzen wurde zunächst der Heißwasserextrakt der Teigprobe 3 Tage gegen dest. Wasser dialysiert (cutoff 12.000 Da.). Anschließend erfolgte im Retentat die Bestimmung der Fructane nach Brandt & Hammes (2001, Getreide Mehl und Brot 55, p341ff).:
Hierzu wurde 0,2 ml der jeweiligen dialysierten Probe mit 0,2 ml Enzymlösung (Sucrase/Maltase, Enzymkit der Fa. Megazyme, Bray, Ireland) im Wasserbad bei 40°C für 30 min inkubiert, und dabei Saccharose und Maltooligosaccharide zu Fructose und Glucose hydrolysiert.
Weiter wurden 0,2 ml alkalische Borhydridlösung (10 mg/ml NaBH4 in 50 mM NaOH) zugesetzt und noch mal 30 min bei 40 °C inkubiert, um die entstanden Zucker zu Zuckeralkoholen zu oxidieren. Danach wurden 0,5 ml Natriumacetat-Puffer (0,1 M) zugefügt. Davon wurde 0,2 ml zusammen mit 0,1 ml Natriumacetat-Lösung (A) und weitere 0,2 ml mit 0,1 ml Fructanase-Lösung (B) in Reagenzgläser gegeben und 20 min bei 40°C inkubiert.
Die entstandene Fructose wird enzymatisch nach Bergmeyer *et al.* (Bergmeyer, H.U., Bernt, E., Schmidt, F. und H. Stork, 1974: Glucose und Fructose, p1241-1246 In: Bergmeyer, H.U (ed) Methoden der enzymatischen Analyse, Verlag Chemie, Weinheim) bestimmt. Die erhaltene Fructosemenge entspricht - nach Abzug des Bindungswassers- dem Fructan (EPS)-Gehalt

Die weiteren Ergebnisse sind in Tabelle 1 aufgeführt.

**Tab.:1 Charakteristik von Teigfermentationen mit Kozakia baliensis**

| Vorteig mit *Kozakia* | Lebendkeimzahl (kbE/ml) *Kozakia baliensis* | [Acetat] (mmol/l) | [Gluconsäure] (mmol/l) | [Exopolysaccharid] (%) |
|---|---|---|---|---|
| 0h | 2,3 x 10⁶ | - | - | 0,55 |
| 24 h | 7,1 x 10⁶ | - | nichts nachgewiesen | 0,54 |
| 48 h | 8,7 x 10⁷ | 28,4 | 10,5 | 0,82 |
| | | | | |
| **Vorteig mit *Kozakia* + 40 g Saccharose** | | | | |
| 0 h | 1,5 x 10⁶ | - | - | 0,47 |
| 24 h | 2,5 x 10⁷ | - | 14,1 | 0,50 |
| 48 h | 1,9 x 10⁸ | 49,0 | 23,0 | 1,12 |

### Beispiel 2: Brot mit Essigsäurebakterien enthaltendem Vorteig hergestellt.

Mit Hilfe des in Beispiel 1 erhaltenem Vorteig (unter Saccharosezusatz) wurden Weizenbrote hergestellt.

900 g Weizenmehl der Type 550, 600 g Wasser, 20 g Salz, 20 g Backhefe sowie 300 g des Essigsäurebakterien enthaltenden Vorteigs (Beispiel 1, 10 % des Mehles) wurden im Spiralkneter in 6 min zu einem Teig verknetet, nach 10 min Ruhe geteilt und aufgemacht. Nach 45 min Stückgare (32 °C, 85 % r.F) wurden die Kästen bei 220 °C für 40 min im Etagenofen gebacken. Nach dem Abkühlen wurde das Volumen bestimmt und in regelmäßigen Abständen die Krumenhärte bestimmt.

Als Vergleichsansätze wurden weitere Brote gebacken, in denen der Teig unter Einsatz eines Vorteiges mit anderen Essigsäurebakterien hergestellt wurde. Hierzu wurde der Vorteig gemäß Beispiel 1, mit Saccarose, angesetzt und zum Einen *Neoasaia chiangmeiensis* und zum Anderen *Gluconobacter frateuri* als Vertreter der Gattung der Essigsäurebakterien gewählt.

Bei allen Ansätzen war eine Verbesserung der Brotstruktur nachweisbar. Tabelle 2 zeigt die Ergebnisse betreffend die Verbesserung der Brotparameter: Volumen und pH-Wert.

**Tabelle 2: Vergleich der Brotparameter**

| | Direkte Führung | Vorteig mit *Neoasaia chiangmeiensis* | Vorteig mit *Kozakia balinensis* | Vorteig mit *Gluconobacter frateuri* |
|---|---|---|---|---|
| Brotvolumen (ml) | 1658 | 1698 | 1730 | 1983 |
| pH | 5,82 | 5,10 | 5,19 | 5,19 |

Die Verbesserung der Brotstruktur ist ferner auch objektiv messbar. Hierzu wird üblicherweise eine Textur Profil Analyse (TPA) und damit eine Charakterisierung der Krumentextur durchgeführt (Figur 1). In Anlehnung an die AACC Methode 74-09 (American Association of Cereals Chemists, St.Paul, Minnesota) werden über einen Penetrationstest mit einem Aluminiumzylinder (Durchmesser 36mm) Krumen-beschreibende Parameter, wie zum Beispiel Krumenhärte (N) und Krumenelastizität (%), bestimmt.

Der Aluminiumstempel fährt in insgesamt zwei Zyklen mit konstanter Geschwindigkeit (0,80 mm/sec) in die Brotscheibe (Dicke: 1,6 mm) und komprimiert diese bis zu einer Verformung von 20% und fährt anschließend wieder aus der Brotscheibe, bis zum Erreichen ihrer Oberfläche, heraus. Die Kraft (N), welche zur Kompression und Re-Kompression der Brotscheibe aufgewendet werden muss, wird gemessen und aufgezeichnet.. Die aufzuwendende Kraft, um die Krume um 20% zu komprimieren ist hier als Krumenhärte definiert.

Figur 1 zeigt hierbei den Verlauf der Krumenhärte über die Lagerdauer von Broten

## Patentansprüche

1. Nicht-entkeimten Getreide- und/oder Pseudogetreidemaische, **dadurch gekennzeichnet, dass** die Maische belüftet ist, mit mindestens einer Spezies aus der Gruppe der Essigsäurebakterien bestehend aus *Gluconobacter,* Gluconacetobacter, *Acidomonas, Kozakia, Neoasaia, Asaia, Swaminathania, Saccharibacter,* sowie *Frateuria* angeimpft wurde und die Maische Stoffwechselprodukte, nämlich Essigsäure, Gluconsäure und Exopolisaccharide gebildet von mindestens einer Spezies aus der Gruppe der Essigsäurebakterien bestehend aus *Gluconobacter,* Gluconacetobacter, *Acidomonas, Kozakia, Neoasaia, Asaia, Swaminathania, Saccharibacter,* sowie *Frateuria* enthält, wobei diese Stoffwechselprodukte aus einen zugesetzten Anteil von 1,5 -10 % Saccharose bezogen auf die eingesetzte Mehlmenge in der Maische gebildet wurden und die Maische eine Konzentration der Essigsäurebakterien mit einer Lebendkeimzahl von 10e8 pro ml aufweist.

2. Nicht-entkeimten Getreide- und/oder Pseudogetreidemaische gemäß Anspruch 1, **dadurch gekennzeichnet, dass** die Maische getrocknet, pasteurisiert oder sterilisiert wird.

3. Verwendung der nicht-entkeimten Getreide- und/oder Pseudogetreidemaischen gemäß Anspruch 1 oder 2 zur *in situ* Produktion von Exopolysaccharide in Getreidemaischen oder Getreideteigen.

4. Verwendung der nicht-entkeimten Getreide- und/oder Pseudogetreidemaischen gemäß Anspruch 1 oder 2 zur Zugabe bei Produktion von Backwaren.

## Claims

1. Non-degerminated cereal mash and/or pseudocereal mash, **characterized in that** the mash is aerated, has been inoculated with at least one species from the group of acetic acid bacteria consisting of *Gluconobacter,* Gluconacetobacter, *Acidomonas, Kozakia, Neoasaia, Asaia, Swaminathania, Saccharibacter* and *Frateuria,* and the mash contains metabolic products, namely acetic acid, gluconic acid and exopolysaccharides formed by at least one species from the group of acetic acid bacteria consisting of *Gluconobacter, Gluconacetobacter, Acidomonas, Kozakia, Neoasaia, Asaia, Swaminathania, Saccharibacter* and *Frateuria,* said metabolic products having been formed from an added proportion of 1.5 - 10% saccharose relative to the employed amount of flour in the mash, and the mash having a concentration of the acetic acid bacteria with a live germ count of 10e8 per ml.

2. The non-degerminated cereal mash and/or pseudocereal mash according to claim 1, **characterized in that** the mash is dried, pasteurized or sterilized.

3. Use of the non-degerminated cereal mashes and/or pseudocereal mashes according to claim 1 or 2 for *in-situ* production of exopolysaccharides in cereal mashes or cereal doughs.

4. Use of the non-degerminated cereal mashes and/or pseudocereal mashes according to claim 1 or 2 for adding during the production of baked goods.

## Revendications

1. Moût de céréales et/ou de pseudo-céréales non dégermées, **caractérisé en ce que** le moût est ventilé, a été inoculé avec au moins une espèce issue du groupe des bactéries d'acide acétique se composant de *Gluconobacter, Gluconacetobacter, Acidomonas, Kozakia, Neoasaia, Asaia, Swaminathania, Saccharibacter,* ainsi que *Frateuria* et **en ce que** le moût contient des métabolites, à savoir de l'acide acétique, de l'acide gluconique et de l'exopolysaccharide constitués par au moins une espèce issue du groupe des bactéries d'acide acétique se composant de *Gluconobacter,* Gluconacetobacter, *Acidomonas, Kozakia, Neoasaia, Asaia, Swaminathania, Saccharibacter,* ainsi que *Frateuria,* sachant que ces métabolites ont été constituées à partir d'un ingrédient additionné de 1,5 à 10 % de saccharose en se référant à la quantité de farine insérée dans le moût et que le moût présente une concentration des bactéries d'acide acétique avec un nombre de germes vivants de 10e8 par ml.

2. Moût de céréales et/ou de pseudo-céréales non dégermées selon la revendication 1, **caractérisé en ce que** le moût est séché, pasteurisé ou stérilisé.

3. Utilisation du moût de céréales et/ou de pseudo-céréales non dégermées selon les revendications 1 ou 2 en vue de la production *in situ* d'exopolysaccharide dans les moûts de céréales ou les pâtes de céréales.

4. Utilisation du moût de céréales et/ou de pseudo-céréales non dégermées selon les revendications 1 ou 2 en vue de l'addition lors de la production de pâtisserie.
